# EUROPEAN PATENT APPLICATION

(11) **EP 1 102 062 A2**
(43) Date of publication of application: **23.05.2001**
(21) Application number: 00203985.7
(22) Date of filing: 14.11.2000
(51) Int. Cl.: G01N 27/22, G01N 33/28

(54) **Method and equipment for detecting stratifications in dispersed water-oil systems**

(30) Priority: 19.11.1999 IT MI992422
(71) Applicant: ENITECNOLOGIE S.p.A., 20097 S. Donato Milanese (Milano) (IT)
(72) Inventor: Di Lullo, Alberto, 25019 Sirmione (Brescia) (IT); Boni, Raffaele, 20097 San Donato Milanese (Milan) (IT)
(74) Representative: De Gregori, Antonella

(57) **Abstract**

Method for detecting stratifications in dispersed oil-water systems consisting in:
- monitoring the electric capacity of a sample of the system of interest comprising at least two phases, along the stratification axis,
- transforming the capacity profile thus produced into the profile of the first derivative of the electric capacity values thus obtained,
- analyzing the resulting curve of the first derivatives.

## Description

The present invention relates to a method for the non- invasive detection and localization of stratifications in dispersed oil-water systems. The term "stratifications" refers to the formation inside the emulsion of regions in which the continuous phase is aqueous and regions in which the continuous phase is oily, which, owing to the difference in density, are situated at different heights inside the container forming well-defined interfaces. Hereinafter, the regions in which the continuous phase is aqueous, will be defined as water-continuous, whereas those in which the continuous phase is oily, will be called oil-continuous.

The method substantially consists in measuring the capacity variations of the system of interest along the stratification axis; the invention itself also relates to the equipment suitable for effecting said method.

The method according to the invention allows the detection of stratifications in systems contained in specific containers made of electrically insulating material and is also effective under total opacity conditions both of the system of interest and container itself.

According to the present invention, the method for detecting stratifications in dispersed oil-water systems comprises the following operations:
- monitoring the electric capacity of a sample of the system of interest comprising at least two phases, along the stratification axis;
- transforming the capacity profile thus produced into the profile of the first derivative of the electric capacity values thus obtained;
- analyzing the resulting curve of the first derivatives with the provision that the following rules can be applied in the analysis of the first derivative:
- if the oil phase has a density lower than that of the aqueous phase (most common case), the interface between an oil-continuous region and a water-continuous region corresponds to a distinct maximum of the capacity first derivative;
- if the oil phase has a density higher than the aqueous phase (less common case), the interface between a water-continuous region and an oil-continuous region corresponds to the passage between a constant derivative zone and an increasing derivative zone.

The method for detecting stratifications in dispersed oil-water systems, effected as described above, particularly when containers made of electrically insulating material are used, allows the desired results to be obtained with the following advantages:
- no physical contact between the emulsion and measurement electrodes;
- transparency of the container and/or dispersed system not necessary;
- possibility of detecting the presence of stratifications, of determining their nature and calculating their position with respect to the stratification axis of the container adopted.

Bearing in mind the general nature of the method for the detection of stratifications in oil-water dispersed systems according to the present invention, the same method can be advantageously effected using equipment, also object of the invention, consisting of the combination of the following elements:
- analog or digital capacitance meter capable of measuring the capacity values within the range of 0.1-10 pF; - automatic system for moving a mobile electrode at a constant rate;
- measurement cell comprising two electrodes, one being fixed and the other mobile.

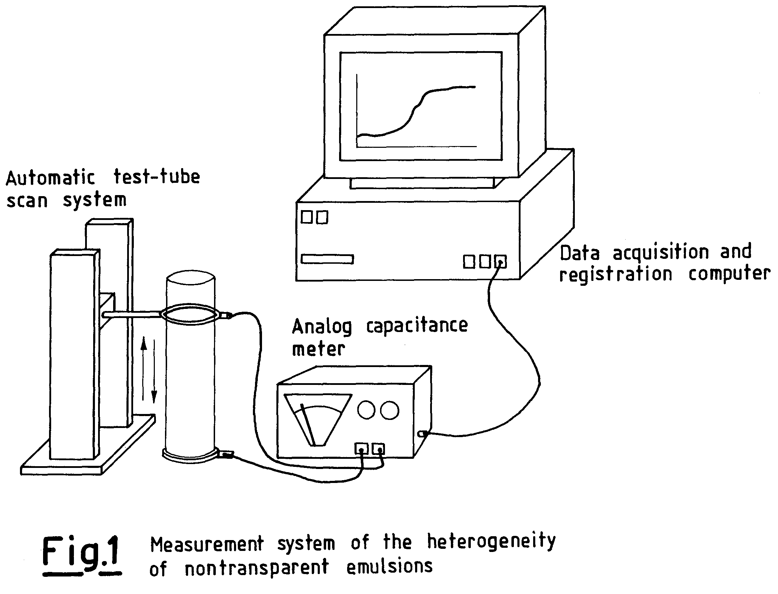

Figure 1 indicates an illustrative scheme of the equipment, object of the present invention. The use of a capacitance meter interfacing a computer equipped with analog-digital acquisition cards and software for the acquisition and registration of the experimental data, has proved to be particularly advantageous.

Analogously, with reference to the measurement cell, another evident advantage lies in the use of circular electrodes, of which one is fixed, situated, for example, near the bottom of the container, and one mobile capable of moving around the container, as close as possible to it, along the stratification axis. The mobile electrode, moreover, is preferably connected to a mechanical system with a motor which allows it to move at a constant rate.

The validity and effectiveness of the method according to the present invention were confirmed by means of a few experimental tests, which provide a better illustration of the invention without limiting its scope.

In the tests, the container consisted of a 40 ml glass test-tube and the following components were used for the formulation of the dispersed systems:
**aqueous phase**
   deionized water with ion exchange resins (milliQ)
   CaCl₂
**oil phase**
   (Lamium/milliQ and Lamium/brine). The relation between the instrumental values and the visible localization values of the interfaces is perfectly linear.
   Lamium (commercial mixture of linear C₈-C₁₃, with an average of C₁₁, paraffinic hydrocarbons); dichloro-methane
**emulsifying agents**
   mixtures with different ratios of the non-ionic surface-active agents Span 20 (sorbitan monolaurate) and Tween 20 (polyethyleneoxide (4) sorbitan monolaurate)

The measurements were effected both on systems consisting of two distinctly separate phases and also on emulsions:
1. Lamium/deionized water interfaces (milliQ) without emulsifying agents
2. Lamium/brine (aqueous solution at 20% of CaCl₂) interfaces without emulsifying agents
3. dichloro-methane/deionized water (milliQ) interfaces without emulsifying agents
4. dichloro-methane/brine (aqueous solution at 10% of CaCl₂) interfaces without emulsifying agents
5. Lamium/brine (aqueous solution at 20% of CaCl₂) emulsions with mixtures at different ratios of Tween 20 and Span 20.

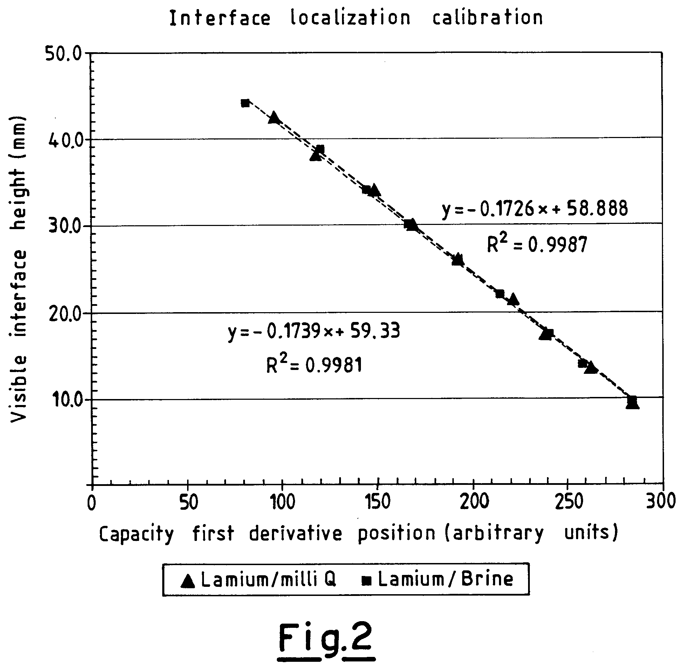

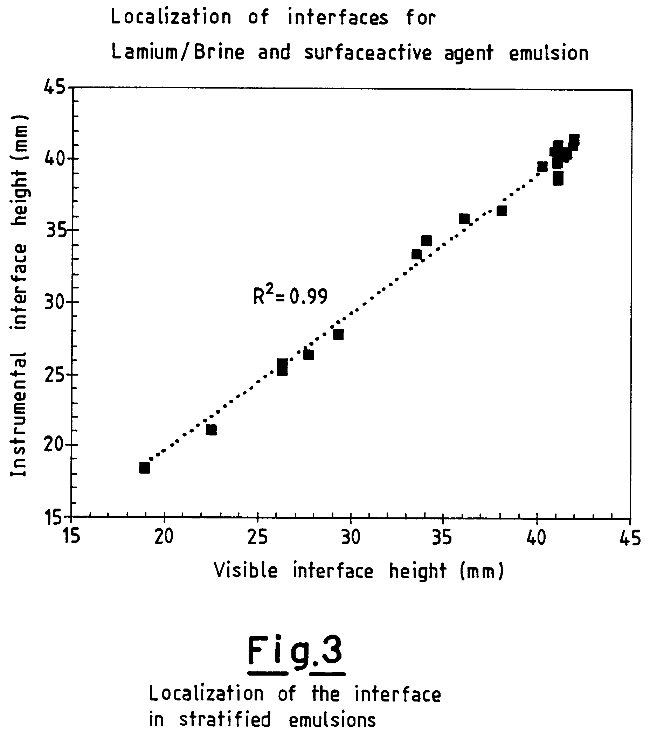

The data analysis is illustrated by the two graphic representations provided in figures 2 and 3 respectively.

Figure 2 indicates the relation between the position of the interfaces instrumentally determined and that measured by direct observation in the systems consisting of two distinctly separate phases (Lamium/milliQ and Lamium/ brine). The relation between the instrumental values and the visible localization values of the interfaces is perfectly linear.

Figure 3 illustrates the relation between the position of the interfaces instrumentally determined and that measured by the direct observation of samples of partially unstable and stratified Lamium/brine emulsions. In this case, the linear correlation index R₂ is slightly lower than that observed for distinctly separate phases (Figure 2), but this can also be attributed to the difficulty in accurately localizing the interfaces visibly, due to the opacity of the phases.

## Claims

1. A method for the detection of stratifications in oil-water dispersed systems consisting in:
- monitoring the electric capacity of a sample of the system of interest comprising at least two phases, along the stratification axis,
- transforming the capacity profile thus produced into the profile of the first derivative of the electric capacity values thus obtained,
- analyzing the resulting curve of the first derivatives.

2. Equipment for effecting the method according to the previous claim consisting of the combination of the following instruments:
- analog or digital capacitance meter capable of measuring the capacity values within the range of 0.1-10 pF;
- automatic system for moving a mobile electrode at a constant rate;
- measurement cell comprising two electrodes, one being fixed and the other mobile.
